# EUROPEAN PATENT APPLICATION

(11) **EP 3 335 703 A1**
(43) Date of publication of application: **20.06.2018**
(21) Application number: 16204721.1
(22) Date of filing: 16.12.2016
(51) Int. Cl.: A61K 9/20, A61K 31/4162, A61P 3/10

(54) **PHARMACEUTICAL COMPOSITION COMPRISING OMARIGLIPTIN**

(71) Applicant: Hexal AG, 83607 Holzkirchen (DE)
(72) Inventor: BORN, Max, D-83607 Holzkirchen (DE); FISCHER-DAUT, Diana, D-83607 Holzkirchen (DE); PUZIK, Andreas, D-83607 Holzkirchen (DE)
(74) Representative: Kluschanzoff, Harald

(57) **Abstract**

The present invention relates to a pharmaceutical composition comprising omarigliptin or a pharmaceutically acceptable salt thereof and less than 75% by weight of neutral excipients, a process for preparing the pharmaceutical composition and its use in the treatment of type 2 diabetes mellitus.

## Description

### FIELD OF THE INVENTION

The present invention relates to a pharmaceutical composition comprising omarigliptin or a pharmaceutically acceptable salt thereof and less than 75% by weight of neutral excipients, a process for preparing the pharmaceutical composition and its use in the treatment of type 2 diabetes mellitus.

### BACKGROUND OF THE INVENTION

Omarigliptin (2R,3S,5R)-2-(2,5-difluorophenyl)-5-[2-(methylsulfonyl)-2,6-dihydropyrrolo[3,4-c]pyrazol-5(4H)-yl]tetrahydro-2H-pyran-3-amine acts as a dipeptidyl peptidase IV (DPP IV) inhibitor and is used for the treatment of type 2 diabetes mellitus.

WO 2010/056708 describes omarigliptin and a pharmaceutical composition comprising 100 mg omarigliptin, 268 mg microcrystalline cellulose, 20 mg croscarmellose sodium, and 4 mg magnesium stearate. Omarigliptin, microcrystalline cellulose and croscarmellose are blended first. The mixture is then lubricated by magnesium stearate and pressed into tablets. Stability of the formulation has not been tested.

WO 2015/031228 describes a stable pharmaceutical composition comprising omarigliptin and neutral excipients in an amount of at least 75% by weight of the pharmaceutical formulation, wherein the neutral excipients comprise at least one non-reducible sugar diluent. The diluent is mannitol or microcrystalline cellulose or a mixture of mannitol and microcrystalline cellulose. Exemplified pharmaceutical formulations are tablets and consist essentially of 15.6% by weight omarigliptin, 59.9% by weight mannitol, 20% by weight microcrystalline cellulose, 2% by weight croscarmellose sodium, and 2.5% by weight magnesium stearate. The tablets are prepared by wet or dry processing methods. Diluents that should not be included in the pharmaceutical formulations described in WO 2015/031228 include lactose monohydrate, polyvinyl pyrrolidone, silicified microcrystalline cellulose, hydroxyethyl cellulose, anhydrous lactose, mannose, glucose, maltose, other reducing sugars, Neusilin (aluminum magnesium metasilicate), Kollidon (polyvinyl pyrrolidone), crospovidone and those with acid functional groups or basic functional groups. It is disclosed that one concern with developing a pharmaceutical composition of omarigliptin is the chemical stability of the compound. Omarigliptin is said to be sensitive to oxidation, hydrolysis (acid and base catalysed) and Maillard degradation (browning) with reducible sugar impurities in excipients.

The pharmaceutical composition of WO 2015/031228 which contains a very high amount of excipients and two different diluents (mannitol and microcrystalline cellulose) has some disadvantages. When a pharmaceutical composition is manufactured, the active ingredient (omarigliptin) has to be homogeneously distributed within the excipients, otherwise, the resulting single dosage forms (such as tablets) do not contain the identical and correct amount of the active ingredient. The higher the amount of the excipients compared to the amount of the active ingredient is, the more difficult or time-consuming it is to achieve sufficient homogeneity. Additionally, the use of two different components as diluents makes the manufacturing process more complex, expensive and time-consuming.

### SUMMARY OF THE INVENTION

It has now been found that stable pharmaceutical compositions comprising omarigliptin can also be obtained by using neutral excipients in an amount of less than 75% by weight of the pharmaceutical formulation.

Accordingly, in a first embodiment of the present invention, it is provided a pharmaceutical composition comprising omarigliptin and neutral excipients, wherein the neutral excipients are present in an amount of less than 75% by weight, preferably less than 70% by weight, more preferably less than 65% by weight.

The pharmaceutical composition of the first embodiment of the present invention has the advantage of a higher active ingredient to excipient ratio which allows to achieve the required homogeneity more easily. Additionally, it has been found that the lower amount of excipients does not influence the bioavailability of the pharmaceutical composition.

A further advantage of the pharmaceutical composition containing a smaller amount of excipients is its smaller size. The size of a pharmaceutical composition, for example a tablet, has an important effect on patient compliance. In particular, in case of omarigliptin which is used in the treatment of type 2 diabetes, a disease prevalent amongst elderly people who may have to take several medicaments and may have swallowing problems, a smaller tablet size may increase patient compliance and, thus, treatment success.

In a second embodiment of the present invention, it is provided a pharmaceutical composition comprising omarigliptin and neutral excipients, wherein the neutral excipients are present in an amount of less than 75% by weight, preferably less than 70% by weight, more preferably less than 65% by weight, and the wherein the neutral excipients comprise mannitol.

It has surprisingly been found that only one diluent may be used without impairing stability or bioavailability of the pharmaceutical composition. By using only one diluent, the process of manufacturing the pharmaceutical composition is simplified, in particular, the step of testing, releasing and adding a further component can be omitted, thus, saving time and production costs, in particular on large-scale industrial production.

### DETAILED DESCRIPTION OF THE INVENTION

The pharmaceutical composition of the present invention comprises omarigliptin or a pharmaceutically acceptable salt thereof, and neutral excipients.

Neutral excipients are pharmaceutically acceptable excipients which do not have an ionic charge. Examples are diluents, such as microcrystalline cellulose and mannitol.

Accordingly, in a preferred embodiment of the present invention, the pharmaceutical composition comprises omarigliptin or a pharmaceutically acceptable salt thereof, and neutral excipients, wherein the neutral excipients are present in an amount of less than 75% by weight, based on the pharmaceutical composition, and the neutral excipients comprise micro-crystalline cellulose.

In another preferred embodiment of the present invention, the pharmaceutical composition comprises omarigliptin or a pharmaceutically acceptable salt thereof, and neutral excipients, wherein the neutral excipients are present in an amount of less than 75% by weight, based on the pharmaceutical composition, and the neutral excipients comprise mannitol.

In another preferred embodiment of the present invention, the pharmaceutical composition comprises omarigliptin or a pharmaceutically acceptable salt thereof, and neutral excipients, wherein the neutral excipients are present in an amount of less than 75% by weight, based on the pharmaceutical composition, and the neutral excipients comprise micro-crystalline cellulose and mannitol.

The pharmaceutical composition according to the present invention may additionally comprise one or more non-neutral excipients. Non-neutral excipients are pharmaceutically acceptable excipients which have an ionic charge, such as salts. The non-neutral excipients include, for example, disintegrants and lubricants.

An exemplified non-neutral disintegrant is croscarmellose sodium.

The non-neutral disintegrant is preferably present in the pharmaceutical composition in an amount of 1 to 15% by weight.

An exemplified non-neutral lubricant is magnesium stearate.

The non-neutral lubricant is preferably present in the pharmaceutical composition in an amount of 1 to 3% by weight, more preferably in an amount of 1.5 to 3% by weight.

In a preferred embodiment, the pharmaceutical composition according to the present invention comprises the non-neutral excipient croscarmellose sodium as disintegrant and the non-neutral excipient magnesium stearate as lubricant.

In the pharmaceutical composition according to the present invention, the neutral excipients are present in an amount of less than 75% by weight, preferably less than 70% by weight, more preferably less than 65% by weight, based on the pharmaceutical composition.

In particular, the neutral excipients are preferably present in the pharmaceutical composition according to the present invention in an amount of more than 50% by weight to less than 75% by weight, more preferably more than 50% by weight to less than 70% by weight, even more preferably more than 50% by weight to less than 65% by weight, based on the pharmaceutical composition.

In a further preferred pharmaceutical composition of the present invention, the combined amount of neutral excipients and non-neutral excipients is less than 85% by weight, preferably less than 80% by weight, more preferably less than 75% by weight, even more preferably less than 70% by weight, based on the pharmaceutical composition.

In particular, the combined amount of neutral excipients and non-neutral excipients in the pharmaceutical composition of the present invention is preferably more than 60% by weight to less than 85% by weight, more preferably more than 60% by weight to less than 80% by weight, even more preferably more than 60% by weight to less than 75% by weight, even more preferably more than 60% by weight to less than 70% by weight, based on the pharmaceutical composition.

Omarigliptin can be present in the pharmaceutical composition of the present invention in the form of a free base or in the form of a pharmaceutically acceptable salt thereof. Pharmaceutically acceptable salts of omarigliptin include, without limitation, the hydrochloride, nitrate, phosphate, sulfate, acetate, benzoate, citrate, malate, maleate, mesylate, besylate, succinate, tartrate, oxalate, lactate, fumarate and mandelate salt. Preferred pharmaceutically acceptable salts of omarigliptin are the hydrochloride, nitrate, phosphate, fumarate and mandelate salt. A particularly preferred pharmaceutically acceptable salts of omarigliptin is the fumarate salt. Another particularly preferred pharmaceutically acceptable salt of omarigliptin is the mandelate salt (including the omarigliptin R-mandelate, omarigliptin S-mandelate and omarigliptin RS-mandelate). Another particularly preferred pharmaceutically acceptable salt of omarigliptin is the hydrochloride salt. The salts can be prepared as known to a skilled person or as described in patent application number EP15201150.8.

Omarigliptin or the pharmaceutically acceptable salt thereof is preferably present in the pharmaceutical composition in an amount of 12.5 mg or 25 mg, calculated as a free base.

Omarigliptin can be prepared as described in WO 2010/056708. Omarigliptin exists in several polymorphic forms and in amorphous form. The pharmaceutical composition according to the present invention may comprise omarigliptin in all polymorphic forms or in amorphous form. Preferably, omarigliptin form I or omarigliptin form V is present in the pharmaceutical composition according to the present invention. The polymorphic forms are described, for example, in WO 2013/003249 and in patent application number PCT/EP2016/069687.

Omarigliptin is preferably present in the pharmaceutical composition according to the present invention in an amount of more than 15% by weight, preferably more than 20% by weight, more preferably more than 25% by weight, even more preferably more than 30% by weight, based on the pharmaceutical composition.

In particular, Omarigliptin is preferably present in the pharmaceutical composition according to the present invention in an amount of more than 15% to less than 40% by weight, preferably more than 20% to less than 40% by weight, more preferably more than 25% to less than 40% by weight, even more preferably more than 30% to less than 40% by weight, based on the pharmaceutical composition.

The pharmaceutical composition according to the present invention is preferably a solid oral dosage form, more preferably a tablet, a capsule, a granulate, pellets or a powder. The tablet may be an uncoated tablet or a coated tablet. In a particularly preferred embodiment, the pharmaceutical composition according to the present invention is a coated tablet. The tablet may have a round, oval or biconvex shape.

The coating preferably comprises hydroxypropyl methylcellulose (HPMC), hydroxypropyl cellulose (HPC) and titanium dioxide. The coating may additionally comprise talc. Optionally, a colorant, for example iron oxide red, iron oxide yellow, or an aluminum lake, may be present in the coating.

Preferably, the pharmaceutical composition of the present invention is obtainable by a process comprising a direct compression step.

In particular, the tablets can be manufactured by first mixing omarigliptin or a pharmaceutically acceptable salt thereof with the excipients, except the magnesium stearate, in a suitable mixer. Optionally, the mixture is then passed through a sieve, for example, through a 1.0 mm sieve. Thereafter, the magnesium stearate is added to the mixture. The thus obtained mixture is then blended in a suitable mixer, for example a Turbula mixer, for 1 to 5 minutes, preferably for about 3 minutes, and then compressed into tablets on a suitable tablet press, for example, a Korsch tablet press.

The tablets can be coated in a suitable coating device, for example, a pan coater or a fluid bed coating device.

The pharmaceutical composition of the present invention can be used in the treatment of metabolic diseases, in particular in the treatment of diabetes mellitus type 2.

Within the meaning of this invention, a pharmaceutical composition is "stable" if less than 0.5% by weight of total impurities are formed under accelerated stability test conditions of 50°C/75% relative humidity in a sealed vial or in an open vial after one week.

### EXAMPLES

The following examples illustrate the present invention and are not intended to limit the scope of the invention set forth in the claims appended hereto.

### Example 1

**Tablet formulation**

| **Component** | **Amount (mg)** | **Amount (% by weight)** | **Amount (mg)** | **Amount (% by weight)** |
|---|---|---|---|---|
| Omarigliptin | 25 | 15.63 | 12.5 | 15.63 |
| Microcrystalline cellulose | 111 | 69.38 | 55.5 | 69.38 |
| Croscarmellose sodium | 20 | 12.50 | 10 | 12.50 |
| Magnesium stearate | 4 | 2.50 | 2 | 2.50 |
| | | | | |
| Sum components | 160 | 100.00 | 80 | 100.00 |
| Sum excipients | 135 | 84.38 | 67.5 | 84.38 |
| Sum neutral excipients | 111 | 69.38 | 55.5 | 69.38 |

All components in the amounts mentioned in the above table, except the magnesium stearate, are mixed and passed through a 1.0 mm sieve. The magnesium stearate in the amount mentioned in the above table is added to the mixture. The thus obtained mixture is blended in a Turbula mixer for 3 minutes and then compressed into round tablets having a diameter of 8 mm (tablets containing 25 mg omarigliptin) or 6 mm (tablets containing 12.5 mg omarigliptin) in a Korsch XP1, 8 OR tablet press.

Bioavailability: The dissolution time of the tablets (n=3) was determined according to the Paddle method described in the European Pharmacopoeia 8.0 chapter 2.9.3 in a test volume of 900 ml in the test medium 0.01 M HCl at a stirring speed of 50 rpm using a Sotax AP 825 device with a photometer (wavelength 228 nm). As a result, more than 90% by weight of the active ingredient omarigliptin was dissolved after 5 min.

### Example 2

**Tablet formulation**

| **Component** | **Amount (mg)** | **Amount (% by weight)** | **Amount (mg)** | **Amount (% by weight)** |
|---|---|---|---|---|
| Omarigliptin | 25 | 31.02 | 12.5 | 31.02 |
| Microcrystalline cellulose | 40 | 49.63 | 20 | 49.63 |
| Mannitol | 12 | 14.89 | 6 | 14.89 |
| Croscarmellose sodium | 1.6 | 1.99 | 0.8 | 1.99 |
| Magnesium stearate | 2 | 2.48 | 1 | 2.48 |
| | | | | |
| Sum components | 80.6 | 100.00 | 40.3 | 100.00 |
| Sum excipients | 55.6 | 68.98 | 27.8 | 68.98 |
| Sum neutral excipients | 52 | 64.52 | 26 | 64.52 |

All components in the amounts mentioned in the above table, except the magnesium stearate, are mixed and passed through a 1.0 mm sieve. The magnesium stearate in the amount mentioned in the above table is added to the mixture. The thus obtained mixture is blended in a Turbula mixer for 3 minutes and then compressed into round tablets having a diameter of 6 mm (tablets containing 25 mg omarigliptin) or 4 mm (tablets containing 12.5 mg omarigliptin) in a Korsch XP1, 8 OR tablet press.

### Example 3

**Tablet formulation**

| **Component** | **Amount (mg)** | **Amount (% by weight)** | **Amount (mg)** | **Amount (% by weight)** |
|---|---|---|---|---|
| Omarigliptin | 25 | 31.02 | 12.5 | 31.02 |
| Microcrystalline cellulose | 39.5 | 49.01 | 19.75 | 49.01 |
| Mannitol | 13.2 | 16.38 | 6.6 | 16.38 |
| Croscarmellose sodium | 1.3 | 1.61 | 0.65 | 1.61 |
| Magnesium stearate | 1.6 | 1.99 | 0.8 | 1.99 |
| | | | | |
| Sum components | 80.6 | 100.00 | 40.3 | 100.00 |
| Sum excipients | 55.6 | 68.98 | 27.8 | 68.98 |
| Sum neutral excipients | 52.7 | 65.38 | 26.35 | 65.38 |

All components in the amounts mentioned in the above table, except the magnesium stearate, are mixed and passed through a 1.0 mm sieve. The magnesium stearate in the amount mentioned in the above table is added to the mixture. The thus obtained mixture is blended in a Turbula mixer for 3 minutes and then compressed into round tablets having a diameter of 6 mm (tablets containing 25 mg omarigliptin) or 4 mm (tablets containing 12.5 mg omarigliptin) in a Korsch XP1, 8 OR tablet press.

Stability: The formulation containing 25 mg omarigliptin was tested for stability under two storage conditions.

| Storage conditions | Total impurities [% by weight] | | |
|---|---|---|---|
| | Initial value | Value after 1 week | Increase |
| 50°C/75% relative humidity in a sealed vial | 0.12 | 0.22 | 0.10 |
| 50°C/75% relative humidity in an open vial | 0.12 | 0.21 | 0.09 |

Bioavailability: The dissolution time of the tablets (n = 3) was determined according to the Paddle method described in the European Pharmacopoeia 8.0 chapter 2.9.3 in a test volume of 900 ml in the test medium 0.01 M HCl at a stirring speed of 50 rpm using a Sotax AP 825 device with a photometer (wavelength 228 nm). As a result, more than 90% by weight of the active ingredient omarigliptin was dissolved after 5 min.

### Example 4

**Tablet formulation**

| **Component** | **Amount (mg)** | **Amount (% by weight)** | **Amount (mg)** | **Amount (% by weight)** |
|---|---|---|---|---|
| Omarigliptin | 25 | 15.78 | 12.5 | 15.78 |
| Mannitol | 109.4 | 69.07 | 54.7 | 69.07 |
| Croscarmellose sodium | 20 | 12.63 | 10 | 12.63 |
| Magnesium stearate | 4 | 2.53 | 2 | 2.53 |
| | | | | |
| Sum components | 158.4 | 100.00 | 79.2 | 100.00 |
| Sum excipients | 133.4 | 84.22 | 66.7 | 84.22 |
| Sum neutral excipients | 109.4 | 69.07 | 54.7 | 69.07 |

All components in the amounts mentioned in the above table, except the magnesium stearate, are mixed and passed through a 1.0 mm sieve. The magnesium stearate in the amount mentioned in the above table is added to the mixture. The thus obtained mixture is blended in a Turbula mixer for 3 minutes and then compressed into round tablets having a diameter of 8 mm (tablets containing 25 mg omarigliptin) or 6 mm (tablets containing 12.5 mg omarigliptin) in a Korsch XP1, 8 OR tablet press.

### Example 5

**Coated tablet formulation**

| **Component** | **Amount (mg)** | **Amount (% by weight)** | **Amount (mg)** | **Amount (% by weight)** |
|---|---|---|---|---|
| Omarigliptin | 25 | 15.63 | 12.5 | 15.63 |
| Microcrystalline cellulose | 111 | 69.38 | 55.5 | 69.38 |
| Croscarmellose sodium | 20 | 12.5 | 10 | 12.5 |
| Magnesium stearate | 4 | 2.5 | 2 | 2.5 |
| | | | | |
| Sum components | 160 | 100 | 80 | 100 |
| Sum excipients | 135 | 84.38 | 67.5 | 84.38 |
| Sum neutral excipients | 111 | 69.38 | 55.5 | 69.38 |

All components in the amounts mentioned in the above table, except the magnesium stearate, are mixed and passed through a 1.0 mm sieve. The magnesium stearate in the amount mentioned in the above table is added to the mixture. The thus obtained mixture is blended in a Turbula mixer for 3 minutes and then compressed into round tablets having a diameter of 8 mm (tablets containing 25 mg omarigliptin) or 6 mm (tablets containing 12.5 mg omarigliptin) in a Korsch XP1, 8 OR tablet press.

The tablets containing 25 mg omarigliptin are coated in a Glatt Minicoater with an aqueous solution of 6.4 mg of the commercially available coating Opadry® Blue 20A99172 (manufactured by Colorcon) which is made of hydroxypropyl methylcellulose (HPMC), hydroxypropyl cellulose (HPC), titanium dioxide, and FD&C Blue #2/Indigocarmine aluminum lake.

The tablets containing 12.5 mg omarigliptin are coated in a Glatt Minicoater with an aqueous solution of 4.8 mg of the commercially available coating Opadry® Yellow 20A92710 (manufactured by Colorcon) which is made of hydroxypropyl methylcellulose (HPMC), hydroxypropyl cellulose (HPC), titanium dioxide, talc, and iron oxide yellow.

### Example 6

**Coated tablet formulation**

| **Component** | **Amount (mg)** | **Amount (% by weight)** | **Amount (mg)** | **Amount (% by weight)** |
|---|---|---|---|---|
| Omarigliptin | 25 | 31.02 | 12.5 | 31.02 |
| Microcrystalline cellulose | 40 | 49.63 | 20 | 49.63 |
| Mannitol | 12 | 14.89 | 6 | 14.89 |
| Croscarmellose sodium | 1.6 | 1.99 | 0.8 | 1.99 |
| Magnesium stearate | 2 | 2.48 | 1 | 2.48 |
| | | | | |
| Sum components | 80.6 | 100.00 | 40.3 | 100.00 |
| Sum excipients | 55.6 | 68.98 | 27.8 | 68.98 |
| Sum neutral excipients | 52 | 64.52 | 26 | 64.52 |

All components in the amounts mentioned in the above table, except the magnesium stearate, are mixed and passed through a 1.0 mm sieve. The magnesium stearate in the amount mentioned in the above table is added to the mixture. The thus obtained mixture is blended in a Turbula mixer for 3 minutes and then compressed into round tablets having a diameter of 6 mm (tablets containing 25 mg omarigliptin) or 4 mm (tablets containing 12.5 mg omarigliptin) in a Korsch XP1, 8 OR tablet press.

The tablets containing 25 mg omarigliptin are coated in a Glatt Minicoater with an aqueous solution of 4.8 mg of the commercially available coating Opadry® Blue 20A99172 (manufactured by Colorcon) which is made of hydroxypropyl methylcellulose (HPMC), hydroxypropyl cellulose (HPC), titanium dioxide, and FD&C Blue #2/Indigocarmine aluminum lake.

The tablets containing 12.5 mg omarigliptin are coated in a Glatt Minicoater with an aqueous solution of 3.6 mg of the commercially available coating Opadry® Yellow 20A92710 (manufactured by Colorcon) which is made of hydroxypropyl methylcellulose (HPMC), hydroxypropyl cellulose (HPC), titanium dioxide, talc, and iron oxide yellow.

### Example 7

**Coated tablet formulation**

| **Component** | **Amount (mg)** | **Amount (% by weight)** | **Amount (mg)** | **Amount (% by weight)** |
|---|---|---|---|---|
| Omarigliptin | 25 | 31.02 | 12.5 | 31.02 |
| Microcrystalline cellulose | 39.5 | 49.01 | 19.75 | 49.01 |
| Mannitol | 13.2 | 16.38 | 6.6 | 16.38 |
| Croscarmellose sodium | 1.3 | 1.61 | 0.65 | 1.61 |
| Magnesium stearate | 1.6 | 1.99 | 0.8 | 1.99 |
| | | | | |
| Sum components | 80.6 | 100.00 | 40.3 | 100.00 |
| Sum excipients | 55.6 | 68.98 | 27.8 | 68.98 |
| Sum neutral excipients | 52.7 | 65.38 | 26.35 | 65.38 |

All components in the amounts mentioned in the above table, except the magnesium stearate, are mixed and passed through a 1.0 mm sieve. The magnesium stearate in the amount mentioned in the above table is added to the mixture. The thus obtained mixture is blended in a Turbula mixer for 3 minutes and then compressed into round tablets having a diameter of 6 mm (tablets containing 25 mg omarigliptin) or 4 mm (tablets containing 12.5 mg omarigliptin) in a Korsch XP1, 8 OR tablet press.

The tablets containing 25 mg omarigliptin are coated in a Glatt Minicoater with an aqueous solution of 4.8 mg of the commercially available coating Opadry® Blue 20A99172 (manufactured by Colorcon) which is made of hydroxypropyl methylcellulose (HPMC), hydroxypropyl cellulose (HPC), titanium dioxide, and FD&C Blue #2/Indigocarmine aluminum lake.

The tablets containing 12.5 mg omarigliptin are coated in a Glatt Minicoater with an aqueous solution of 3.6 mg of the commercially available coating Opadry® Yellow 20A92710 (manufactured by Colorcon) which is made of hydroxypropyl methylcellulose (HPMC), hydroxypropyl cellulose (HPC), titanium dioxide, talc, and iron oxide yellow.

### Example 8

**Coated tablet formulation**

| **Component** | **Amount (mg)** | **Amount (% by weight)** | **Amount (mg)** | **Amount (% by weight)** |
|---|---|---|---|---|
| Omarigliptin | 25 | 15.78 | 12.5 | 15.78 |
| Mannitol | 109.4 | 69.07 | 54.7 | 69.07 |
| Croscarmellose sodium | 20 | 12.63 | 10 | 12.63 |
| Magnesium stearate | 4 | 2.53 | 2 | 2.53 |
| | | | | |
| Sum components | 158.4 | 100.00 | 79.2 | 100.00 |
| Sum excipients | 133.4 | 84.22 | 66.7 | 84.22 |
| Sum neutral excipients | 109.4 | 69.07 | 54.7 | 69.07 |

All components in the amounts mentioned in the above table, except the magnesium stearate, are mixed and passed through a 1.0 mm sieve. The magnesium stearate in the amount mentioned in the above table is added to the mixture. The thus obtained mixture is blended in a Turbula mixer for 3 minutes and then compressed into round tablets having a diameter of 8 mm (tablets containing 25 mg omarigliptin) or 6 mm (tablets containing 12.5 mg omarigliptin) in a Korsch XP1, 8 OR tablet press.

The tablets containing 25 mg omarigliptin are coated in a Glatt Minicoater with an aqueous solution of 6.4 mg of the commercially available coating Opadry® Blue 20A99172 (manufactured by Colorcon) which is made of hydroxypropyl methylcellulose (HPMC), hydroxypropyl cellulose (HPC), titanium dioxide, and FD&C Blue #2/Indigocarmine aluminum lake.

The tablets containing 12.5 mg omarigliptin are coated in a Glatt Minicoater with an aqueous solution of 4.8 mg of the commercially available coating Opadry® Yellow 20A92710 (manufactured by Colorcon) which is made of hydroxypropyl methylcellulose (HPMC), hydroxypropyl cellulose (HPC), titanium dioxide, talc, and iron oxide yellow.

### Comparative Example 1

**Coated tablet formulation according to WO2015/031228**

| **Component** | **Amount (mg)** | **Amount (% by weight)** | **Amount (mg)** | **Amount (% by weight)** |
|---|---|---|---|---|
| Omarigliptin | 25 | 15.63 | 12.5 | 15.63 |
| Microcrystalline cellulose | 32 | 20.00 | 16 | 20.00 |
| Mannitol | 95.8 | 59.88 | 47.9 | 59.88 |
| Croscarmellose sodium | 3.2 | 2.00 | 1.6 | 2.00 |
| Magnesium stearate | 4 | 2.50 | 2 | 2.50 |
| | | | | |
| Sum components | 160 | 100.00 | 80 | 100.00 |
| Sum excipients | | 84.38 | | 84.38 |
| Sum neutral excipients | | 79.88 | | 79.88 |
| | | | | |
| Opadry Yellow 20A92710* | | | 4.8 | |
| Opadry Blue 20A99172** | 6.4 | | | |

All components in the amounts mentioned in the above table, except the magnesium stearate, are mixed and passed through a 1.0 mm sieve. The magnesium stearate in the amount mentioned in the above table is added to the mixture. The thus obtained mixture is blended in a Turbula mixer for 3 minutes and then compressed into round tablets having a diameter of 8 mm (tablets containing 25 mg omarigliptin) or 6 mm (tablets containing 12.5 mg omarigliptin) in a Korsch XP1, 8 OR tablet press.

The tablets containing 25 mg omarigliptin are coated in a Glatt Minicoater with an aqueous solution of 6.4 mg of the commercially available coating Opadry® Blue 20A99172 (manufactured by Colorcon) which is made of hydroxypropyl methylcellulose (HPMC), hydroxypropyl cellulose (HPC), titanium dioxide, and FD&C Blue #2/Indigocarmine aluminum lake.

The tablets containing 12.5 mg omarigliptin are coated in a Glatt Minicoater with an aqueous solution of 4.8 mg of the commercially available coating Opadry® Yellow 20A92710 (manufactured by Colorcon) which is made of hydroxypropyl methylcellulose (HPMC), hydroxypropyl cellulose (HPC), titanium dioxide, talc, and iron oxide yellow.

Stability: The formulation containing 12.5 mg omarigliptin was tested for stability under two storage conditions:

| Storage conditions | Total impurities [% by weight] | | |
|---|---|---|---|
| | Initial value | Value after 1 week | Increase |
| 50°C/75% relative humidity in a sealed vial | 0.00 | 0.10 | 0.10 |
| 50°C/75% relative humidity in an open vial | 0.00 | 0.10 | 0.10 |

Bioavailability: The dissolution time of the tablets (n = 3) was determined according to the Paddle method described in the European Pharmacopoeia 8.0 chapter 2.9.3 in a test volume of 900 ml in the test medium 0.01 M HCl at a stirring speed of 50 rpm using a Sotax AP 825 device with a photometer (wavelength 228 nm). As a result, more than 90% by weight of the active ingredient omarigliptin was dissolved after 5 min.

## Claims

1. A pharmaceutical composition comprising
omarigliptin or a pharmaceutically acceptable salt thereof, and
neutral excipients,
wherein the neutral excipients are present in an amount of less than 75% by weight, based on the pharmaceutical composition.

2. Pharmaceutical composition according to claim 1, wherein the neutral excipients comprise microcrystalline cellulose.

3. Pharmaceutical composition according to claim 1, wherein the neutral excipients comprise mannitol.

4. Pharmaceutical composition according to claim 1, wherein the neutral excipients comprise microcrystalline cellulose and mannitol.

5. Pharmaceutical composition according to any of claims 1 to 4, wherein omarigliptin or a pharmaceutically acceptable salt thereof is present in an amount of 12.5 mg or 25 mg, calculated as a free base.

6. Pharmaceutical composition according to any of claims 1 to 5 additionally comprising a non-neutral excipient.

7. Pharmaceutical composition according to claim 6, wherein the non-neutral excipient comprises a disintegrant and/or a lubricant.

8. Pharmaceutical composition according to claim 7, wherein the disintegrant is croscarmellose sodium.

9. Pharmaceutical composition according to claim 7, wherein the lubricant is magnesium stearate.

10. Pharmaceutical composition according to any of claims 1 to 9, wherein the neutral excipients are present in an amount of less than 70% by weight, preferably in an amount of less than 65% by weight, based on the pharmaceutical composition.

11. Pharmaceutical composition according to any of claims 1 to 10, wherein the combined amount of neutral excipients and non-neutral excipients is less than 85% by weight, preferably less than 80% by weight, more preferably less than 75% by weight, even more preferably less than 70% by weight, based on the pharmaceutical composition.

12. Pharmaceutical composition according to any of claims 1 to 11 which is a solid oral dosage form, preferably a tablet, a capsule, a granulate, pellets or a powder.

13. Pharmaceutical composition according to claim 12 which is a tablet, preferably an uncoated tablet or a coated tablet.

14. Pharmaceutical composition according to claim 13 obtainable by a process comprising a direct compression step.

15. Pharmaceutical composition according to any of claims 1 to 14 for use in the treatment of diabetes mellitus type 2.
